# EUROPÄISCHE PATENTSCHRIFT

(11) **EP 2 359 126 B1**
(45) Veröffentlichungstag und Bekanntmachung des Hinweises auf die Patenterteilung: **03.08.2016**
(21) Anmeldenummer: 09801914.4
(22) Anmeldetag: 27.10.2009
(51) Int. Cl.: D04B 9/14, G01N 27/24, G01N 27/22, G01N 33/36

(54) **SENSOREINHEIT ZUR ÜBERWACHUNG DER QUALITÄT VON FASERMATERIAL**
SENSOR UNIT FOR MONITORING QUALITY OF FIBROUS MATERIAL
UNITÉ DE DÉTECTION POUR CONTRÔLER DE QUALITÉ DE MATÉRIAU FIBREUX

(30) Priorität: 25.11.2008 DE 102008059176
(43) Veröffentlichungstag der Anmeldung: 24.08.2011
(73) Patentinhaber: SIPRA Patententwicklungs- und Beteiligungsgesellschaft mbH, 72438 Albstadt (DE)
(72) Erfinder: FLAD, Axel, 72393 Burladingen (DE); SCHWAB, Manuel, 72127 Kusterdingen (DE)
(74) Vertreter: Kohler Schmid Möbus Patentanwälte
(86) Internationale Anmeldenummer: PCT/DE2009/001477
(87) Internationale Veröffentlichungsnummer: WO 2010/060399

(56) Entgegenhaltungen:
- EP-A1- 0 924 518
- WO-A1-2006/105676
- DE-A1- 19 908 236
- US-A- 3 754 172

## Beschreibung

Die Erfindung betrifft eine Sensoreinheit der im Oberbegriff des Anspruchs 1 angegebenen Gattung und eine mit einer solchen Sensoreinheit ausgerüstete Maschine zur Herstellung von Maschenware.

In der Textilindustrie und hier insbesondere in der Spinnereitechnik ist es allgemein üblich, das zu verarbeitende Fasermaterial laufend auf seine Qualität zu überwachen. Unter "Fasermaterial" werden im Rahmen der vorliegenden Erfindung im Wesentlichen lineare, lang gestreckte, textile Materialien, insbesondere Faserbänder, Garne, Lunten, Flyerlunten, Filamente usw. verstanden, die einem bestimmten Verarbeitungsprozess unterworfen und zu diesem Zweck einem Streckwerk, einer Spinnvorrichtung od. dgl. zugeführt werden sollen.

Die Qualitätsüberwachung derartiger Fasermaterialien kann z. B. mit mechanischen, optischen oder kapazitiven Mitteln erfolgen. Für eine mechanische Überwachung werden Sensoreinheiten verwendet, die eine Rolle mit einer zur Aufnahme des Fasermaterials bestimmten Umfangsnut aufweisen, in die z. B. eine Tastrolle (DE 28 50 775 A1) oder ein Tastfinger (DE 199 50 901 A1) eingreift. Schwankungen in der Dichte oder Masse des Fasermaterials haben entsprechende Schwankungen der Lage der Tastrolle oder des Tastfingers zur Folge und werden mit diesen zugeordneten Wegmessern erfasst. Optische Sensoreinheiten (z. B. DE 32 37 371 A1) weisen aus Lumineszenzdioden und Phototransistoren gebildete Photozellen auf, die vom Fasermaterial durchlaufen werden. Kapazitiv arbeitende Sensoreinheiten schließlich sind mit einem Messkondensator versehen (z. B. EP 0 924 513 A1, DE 199 08 236 A1), der zwei parallel angeordnete Kondensatorplatten aufweist, die einen vom Fasermaterial durchlaufenen Durchgang begrenzen. Als Messgröße dient hier die Kapazität des Messkondensators, die sich entsprechend den Schwankungen im Fasermaterial verändert.

Sensoreinheiten der beschriebenen Art werden neuerdings auch für so genannte Spinnstrickmaschinen (z. B. PCT WO 2004/079068 A2) und andere maschenbildende Maschinen benötigt, denen anstelle eines üblichen Garns unmittelbar ein in einem Streckwerk oder auf andere Weise verfeinertes Fasermaterial in Form eines Faserbandes, einer Flyerlunte od. dgl. zugeführt wird. Mechanische Sensoreinheiten könnten hier mit Vorteil eingesetzt werden, da sie ein vergleichsweise geringes Signalrauschen verursachen, weitgehend unempfindlich gegen äußere Einflüsse sind und Schwankungen in der Fasermenge (z. B. Zahl der Fasern im Querschnitt eines Fasermaterials) mit ausreichender Genauigkeit abbilden. Ein Nachteil besteht allerdings darin, dass sie aus wenigstens zwei Komponenten, nämlich einem beweglichen Bauteil (Tastrolle, Tastfinger od. dgl.) und einem elektrischen Wegmesser bestehen. Da moderne Strickmaschinen z. B. 96 Stricksysteme aufweisen können und pro Stricksystem wenigstens je eine oder mehrere Sensoreinheiten benötigt werden, wären die allein für die Überwachung des Fasermaterials aufzuwendenden Kosten unvertretbar hoch.

Optische Sensoreinheiten sind für die Zwecke einer permanenten Überwachung von Fasermaterial an Spinnstrickmaschinen weitgehend ungeeignet, da sie einerseits zu ungenau arbeiten und andererseits empfindlich gegenüber unvermeidbaren Verunreinigungen sind.

Da die Anwendung von Plattenkondensatoren in der Regel nicht ausreichend genau ist und z. B. durch unvermeidbares Signalrauschen gestört wird, sind auch bereits kapazitiv arbeitende Sensoreinheiten der eingangs bezeichneten Gattung bekannt geworden (z. B. PCT WO 2006/105676 A1), bei denen eine der beiden Kondensatorplatten an ihren Rändern mit zwei isoliert angebrachten Schutzelektroden versehen ist, an die eine sich zeitlich verändernde Spannung angelegt wird. Sensoreinheiten dieser Art haben zwar den Vorteil, dass sie preisgünstig herstellbar sind. Sie besitzen jedoch den Nachteil, dass sie über keine mechanische Führung für das Fasermaterial verfügen und die Messsignale eine zu geringe, kaum über ein Grundrauschen hinaus gehende Signalstärke haben, so dass nicht tolerierbare Schwankungen der Faserdichte oder Fasermasse nur ungenau ermittelt werden können. Das gilt zumindest für ihre An-wendung in Maschinen zur Herstellung von Maschenware. Da die Faserdicke und/oder die auf eine Einheitslänge bezogene Fasermasse der auf den Markt angebotenen Fasermaterialien - in deren Längs- bzw. Transportrichtung betrachtet - vergleichsweise häufigen Schwankungen unterworfen sind, müssen bisher entweder Maschenwaren mit ungleichförmiger Qualität, die durch Dick- oder Dünnstellen im Fasermaterial verursacht wird, in Kauf genommen oder aufwendige Maßnahmen getroffen werden, um derartige Schwankungen im Fasermaterial zu vermeiden oder die mit Fehlstellen versehenen Bereiche aus der Maschenware zu entfernen.

Zur Vermeidung dieses Nachteils ist in einer noch unveröffentlichen Patentanmeldung derselben Anmelderin eine Sensoreinheit der eingangs bezeichneten Gattung vorgeschlagen worden, die eine Elektrodenanordnung aufweist, die sich insbesondere für die Anwendung des sogenannten 3-Elektroden Messprinzips eignet. Messungen haben gezeigt, dass mit einer derartiger Elektrodenanordnung selbst dann, wenn sehr kleine kapazitive Änderungen vorhanden sind, vergleichsweise hohe Messsignalpegel erhalten werden. Aufgrund der Gestaltung der Messelektrode wird außerdem eine so gleichmäßige Zwangsführung des Fasermaterials erzielt, dass das vom Fasermaterial verursachte, unvermeidbare Signalrauschen nicht durch unkontrollierte Bewegungen das Fasermaterials im Messbereich vergrößert wird. Dadurch lassen sich durch Fasermasseschwankungen, Fremdkörpereinschlüsse od. dgl. verursachte Dick- oder Dünnstellen im Fasermaterial gut erkennen. Beim praktischen Gebrauch dieser Sensoreinheit hat sich allerdings herausgestellt, dass sie gegenüber äußeren Einflüssen empfindlich ist, der Aufbau ihrer Elektrodenanordnung die Gefahr mit sich bringt, dass sich lose Fasern in ihr einklemmen, und sowohl die Fertigung als auch die Montage der verschiedenen Bauteile nicht befriedigt.

Ausgehend davon liegt der Erfindung das technische Problem zugrunde, die eingangs bezeichnete Sensoreinheit so auszubilden, dass sie zu vergleichsweise starken Messsignalen führt, gegen äußere Störungen weitgehend unempfindlich ist und dennoch preisgünstig hergestellt und montiert werden kann. Außerdem soll eine mit einer solchen Sensoreinheit ausgerüstete Maschine geschaffen werden.

Gelöst wird dieses Problem durch eine Sensoreinheit mit den kennzeichnenden Merkmalen des Anspruchs 1 und eine Maschine mit den kennzeichnenden Merkmalen des Anspruchs 27.

Die Erfindung bringt den Vorteil mit sich, dass die Gleitfläche für das Fasermaterial nur noch aus einer Komponente bestehen braucht und daher das Einklemmen von Fasern vermieden wird. Außerdem wird eine gute Abschirmung der Messelektrode und daher eine geringe Empfindlichkeit gegen äußere Störungen erreicht. Schließlich bietet die erfindungsgemäße Sensoreinheit Vorteile im Hinblick auf die Fertigung, den Zusammenbau der verschiedenen Teile und ihre Montage an einer zur Herstellung von Maschenware bestimmten Maschine.

Weitere vorteilhafte Merkmale der Erfindung ergeben sich aus den Unteransprüchen.

Die Erfindung wird nachfolgend in Verbindung mit den beiliegenden Zeichnungen an Ausführungsbeispielen näher erläutert. Es zeigen:
Fig. 1 eine schematische Seitenansicht einer erfindungsgemäßen Sensoreinheit;
Fig. 2 einen schematischen Schnitt durch die Sensoreinheit etwa längs einer Linie II-II der Fig. 1;
Fig. 3 eine Unteransicht eines Sensorkopfs der Sensoreinheit nach Fig. 2;
Fig. 4 einen Längsschnitt durch eine Sensorbank mit vier entsprechend Fig. 1 bis 3 ausgebildeten Sensoreinheiten;
Fig. 5 schematisch einen Schnitt längs der Linie V - V der Fig. 4; und
Fig. 6 eine Rundstrickmaschine mit einer erfindungsgemäßen Sensoreinheit.

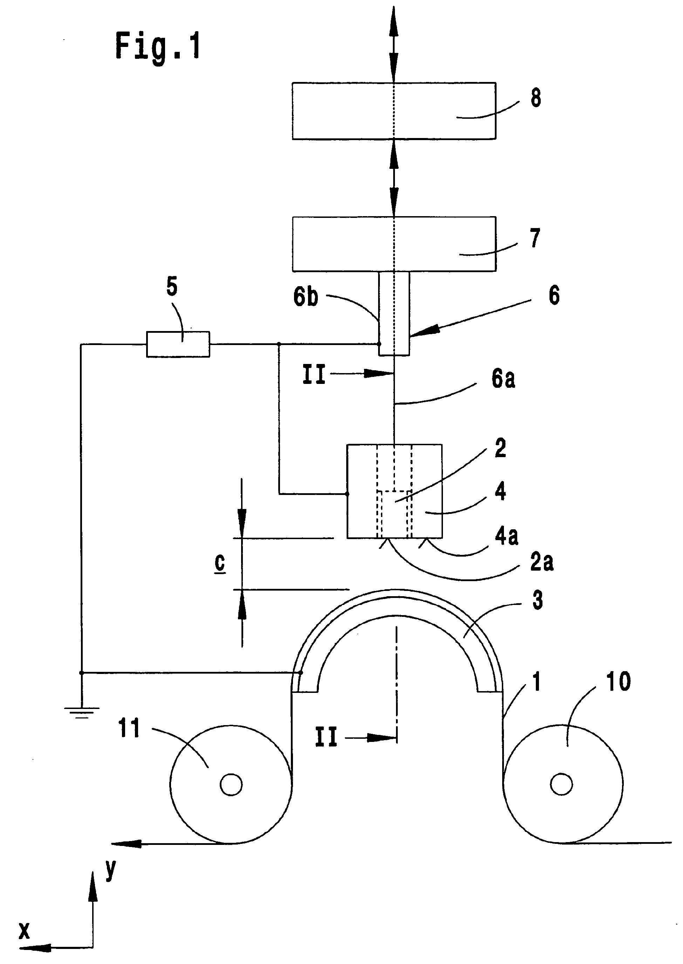

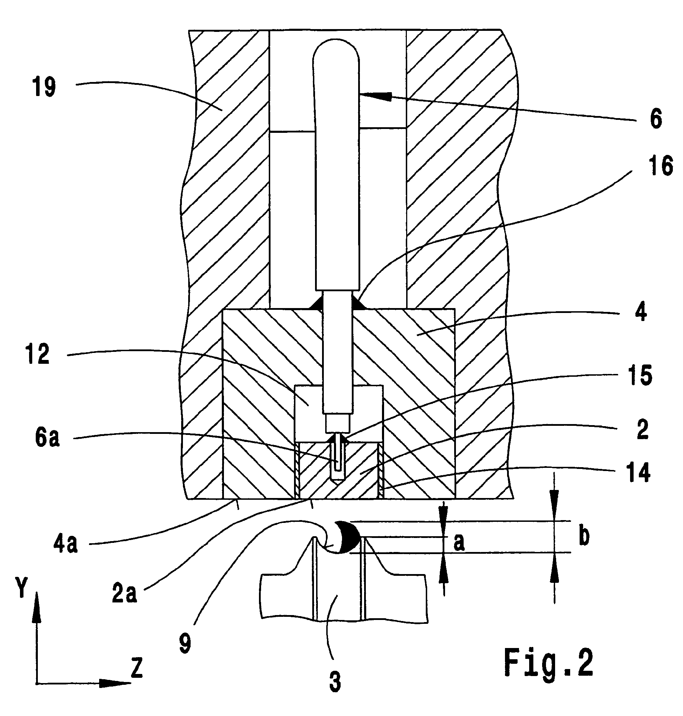

Fig. 1 und 2 zeigen eine erfindungsgemäße, kapazitiv arbeitende Sensoreinheit, die zur Überwachung der Qualität eines als Faserband, Flyerlunte od. dgl. vorliegenden Fasermaterials 1 dient, das in einem kontinuierlichen Prozess z. B. einem nicht dargestellten Streckwerk zugeführt wird. Das Fasermaterial 1 soll z. B. auf seine Homogenität und in diesem Zusammenhang insbesondere auf das Vorhandensein von Dick- oder Dünnstellen überprüft werden, die z. B. durch nicht gleichförmige Fasermassen pro Einheitslänge, eine unterschiedliche Anzahl von Fasern im Querschnitt des Fasermaterials 1 oder auch durch Fremdkörpereinschlüsse im Fasermaterial od. dgl. verursacht sein können. Ungleichmäßigkeiten dieser Art werden nachfolgend allgemein als "Fehlstellen" im Fasermaterial bezeichnet.

Die erfindungsgemäße Sensoreinheit arbeitet im Gegensatz zu bekannten Sensoreinheiten nach dem kapazitiven 3-Elektroden-Messprinzip. Sie enthält zu diesem Zweck eine Elektrodenanordnung, die eine erste, als Messelektrode 2 ausgebildete Elektrode, eine nachfolgend als Sendeelektrode 3 bezeichnete, zweite Elektrode und eine nachfolgend als Schirmelektrode 4 bezeichnete, dritte Elektrode aufweist. Alle drei Elektroden bestehen aus einem elektrisch gut leitenden Material, insbesondere Metall, und können als Kondensatorelektroden aufgefasst werden. Wie in Fig. 1 schematisch angedeutet ist, ist die Messelektrode 2 gegenüber der Sendeelektrode 3 angeordnet. Die Sendeelektrode 3 ist mit dem Signalausgang eines eine Wechselspannung von z.B. ca. 20 kHz liefernden Generators 5 verbunden, dessen anderer Anschluss an die Schirmelektrode 4 angeschlossen ist und auf einem virtuellen Nullpotential liegt. Zwischen der Messelektrode 2 und der Sendeelektrode 3 befindet sich ein Durchgang für das Fasermaterial 1. Außerdem ist die Messelektrode 2 mit dem Innenleiter 6a eines Koaxialkabels 6 verbunden, dessen Außenleiter 6b mit der Schirmelektrode 4 verbunden ist und daher auf demselben Potential wie diese liegt. Der Innenleiter 6a ist an einen Verstärker 7 angeschlossen, dessen Ausgang mit einer Auswerteelektronik 8 verbunden ist, die außerdem das virtuelle Nullpotential für die Schirmelektrode 4 liefert. Die Sendeelektrode 3 wird zusätzlich mit dem als Maschinenmasse bezeichneten Potential verbunden, das z. B. dem Massenpotential einer Rundstrickmaschine, eines Streckwerks od. dgl. entspricht, denen das zu untersuchende Fasermaterial 1 zugeführt wird.

Eine für die Zwecke der Erfindung als besonders wesentlich erachtete Voraussetzung zur Erzielung sicherer Messergebnisse ist eine gleichmäßige Führung des Fasermaterials 1 durch die Sensoreinheit. Erfindungsgemäß ist daher vorgesehen, der Sendeelektrode 3 die aus Fig. 1 und 2 ersichtliche Form zu geben. Wird der Einfachheit halber angenommen, dass das Fasermaterial 1 in Richtung der X-Achse eines gedachten, kartesischen Koordinatensystems bewegt wird, die Elektroden 2 und 3 entsprechend Fig. 1 in Richtung der Y-Achse des gedachten Koordinatensystems beabstandet sind und dessen Z-Achse in Fig. 1 senkrecht zur Zeichenebene verläuft, dann ist die Sendeelektrode 3 zunächst so gestaltet, dass sie in der Transportrichtung X einen bogenförmigen, z. B. etwa halbkreisförmigen Verlauf hat und mit ihrer Längsachse parallel zur XY-Ebene angeordnet ist. Außerdem ist die Sendeelektrode 3 so angeordnet, dass ihr konvex gewölbtes Teil eine der Messelektrode 2 zugewandte Oberseite bildet, wie aus Fig. 1 und 2 klar ersichtlich ist. Quer zur bogenförmigen Krümmung, d. h. in der YZ-Ebene gemäß Fig. 2, besitzt die Sendeelektrode 3 dagegen einen muldenförmigen Verlauf. Eine dadurch gebildete, zur Messelektrode 2 hin offene Mulde bildet eine stationäre Gleitfläche 9, längs welcher das Fasermaterial 1 durch die Sensoreinheit gleitet. Vorzugsweise ist die Gleitfläche 9 im Wesentlichen halbzylindrisch ausgebildet, wobei ihr Radius zweckmäßig geringfügig größer als der Durchmesser des Fasermaterials 1 ist. Insgesamt hat die Sendeelektrode 3 daher das Aussehen einer zur Messelektrode 2 hin offenen Dachrinne (Fig. 2), die in ihrer Längsrichtung, d. h. in Transportrichtung X, zusätzlich in der aus Fig. 1 ersichtlichen Weise bogenförmig gekrümmt ist.

Durch die beschriebene Form der Gleitfläche 9 wird eine gute seitliche Führung des Fasermaterials 1 sichergestellt, das in Fig. 2 mit einem kreisförmigen Querschnitt dargestellt ist, aber auch andere, z. B. ovale Querschnitte besitzen kann.

Damit das Fasermaterial 1 längs der gesamten Sendeelektrode 3 gleichförmig an der Gleitfläche 9 anliegt, ist an einer Eintrittsseite der Sensoreinheit ein Niederhalter 10 und an der Austrittsseite der Sensoreinheit ein Niederhalter 11 angeordnet. Die Niederhalter 10 und 11 sind bevorzugt als Umlenkelemente ausgebildet, deren Ausgangs- bzw. Eingangsflächen in den Verlängerungen der Sendeelektrode 3 liegen, damit das Fasermaterial 1 der Sendeelektrode 3 tangential zugeführt und auch tangential wieder von ihr entfernt wird, wie Fig. 1 zeigt. Dadurch wird einerseits das Fasermaterial 1 einseitig fest gegen die Gleitfläche 9 gedrückt, während andererseits vermieden wird, dass das Fasermaterial 1 während des Durchgangs durch die Sensoreinheit zu stark umgelenkt oder geknickt wird. Störungen der auch vom Abstand des Fasermaterials 1 von der Gleitfläche 9 abhängigen Messsignale aufgrund einer ungleichförmigen Führung oder Anlage des Fasermaterials 1 auf der Gleitfläche 9 werden dadurch sicher vermieden.

Da erfahrungsgemäß die in Transportrichtung X gemessenen Längen üblicher Fehlstellen nur ca. 10 mm bis 20 mm betragen, sollte die X-Richtung gemessene Bogenlänge der Sendeelektrode 3 einerseits wenigstens etwa genauso groß, andererseits aber auch nicht wesentlich größer sein und höchstens z. B. 40 mm betragen. Um auch kleinste Fehlstellen zu entdecken, wird das Verhältnis Fehlstellenlänge zur Sendeelektrodenlänge vorzugsweise zwischen 1 : 1 und 1 : 4 gewählt. Außerdem sollte die in Y-Richtung gemessene Tiefe a (Fig. 2) der muldenförmigen Gleitfläche 9 höchstens gleich dem Durchmesser b des Fasermaterials 1, vorzugsweise jedoch höchstens halb so groß wie dieser sein. Dadurch werden ein günstiger Verlauf der elektrischen Feldlinien im Fasermaterial 1 und ein vergleichsweise starkes Messsignal erhalten, ohne dass die seitliche Führung des Fasermaterials 1 verloren geht.

Die Gleitfläche 9 und/oder mit dem Fasermaterial in Berührung kommende Flächen der Niederhalter 10, 11 können aus einem Material mit geringem Gleitwiderstand bestehen oder bei Bedarf mit einem den Gleitwiderstand reduzierenden Material beschichtet sein. Vorzugsweise bestehen die Niederhalter 10, 11 aus fest stehenden Umlenkelementen mit einem niedrigen Reibwert. Alternativ können die Niederhalter 10, 11 zur Verbesserung des Transports des Fasermaterials 1 aber auch als frei drehbar gelagerte Rollen ausgebildet sein oder aus angetriebenen Rollen bestehen und mit nicht dargestellten Antriebsmitteln verbunden sein, um sie während des Betriebs mit gleichen oder unterschiedlichen Umfangsgeschwindigkeiten in Umdrehungen zu versetzen.

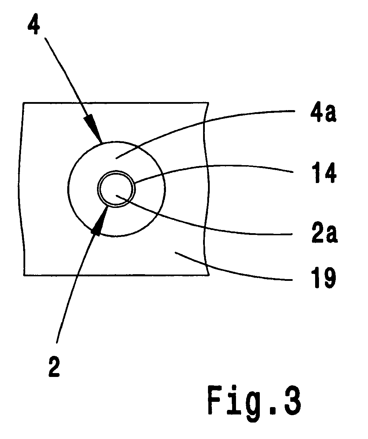

Die Messelektrode 2 ist, wie insbesondere Fig. 2 und 3 zeigen, vorzugsweise im Wesentlichen zylindrisch ausgebildet und mit ihrer Achse in y-Richtung erstreckt (vgl. auch Fig. 1). Außerdem besitzt die Messelektrode 2 eine Messfläche 2a, die der Oberseite, vorzugsweise dem höchsten Punkt der Oberseite der Sendeelektrode 3 mit einem Abstand c (Fig. 1) gegenübersteht, vorzugsweise eben ausgebildet ist und eine Parallele zur xz-Ebene des gedachten Koordinatensystems bildet.

Die Schirmelektrode 4 ist so ausgebildet, dass sie die Messelektrode 2 mit Ausnahme von deren Messfläche 2a allseitig umschließt. Zu diesem Zweck ist die Schirmelektrode 4 vorzugsweise als ein zylindrischer Topf ausgebildet und mit einem ebenfalls zylindrischen, zur Sendeelektrode 3 hin offenen Aufnahmeraum 12 (Fig. 2) versehen, in dem die Messelektrode 2 koaxial angeordnet ist, wie insbesondere Fig. 3 zeigt. Zwischen der äußeren Mantelfläche der Messelektrode 2 und der den Aufnahmeraum 12 begrenzenden Innenwand der Schirmelektrode 4 ist eine rundum erstreckte, z. B. aus Kunststoff bestehende Isolationsschicht 14 angebracht, wodurch die Schirmelektrode 4 elektrisch von der Messelektrode 2 isoliert ist.

Eine ringförmige, mit der Messelektrode 2 koaxiale Stirnfläche 4a, die die topfförmige Schirmelektrode 4 auf ihrer der Sendeelektrode 3 zugewandten Seite abschließt, ist vorzugsweise eben ausgebildet und mit der Messfläche 2a in einer und derselben Ebene angeordnet. Dadurch wird der Vorteil erzielt, dass die Messelektrode 2 und die Schirmelektrode 4 einen Sensorkopf bilden, der nicht nur auf einfache Weise als vorgefertigte Baueinheit hergestellt werden kann, sondern in dem auch die Messelektrode 2 bis auf die dem Fasermaterial 1 zugewandte Messfläche 2a rundum durch die Schirmelektrode 4 nach außen gegen störende Felder abgeschirmt ist. Außerdem steht die auf dem Potential der Maschinenmasse befindliche Sendeelektrode 3 der Messfläche 2a vorzugsweise mit einem vergleichsweise geringen Abstand c gegenüber, wodurch sie die einzige noch freie Seite der Messelektrode 2 nach außen abschirmt. Das ist besonders bei Anwendung der Sensoreinheit an einer Rundstrickmaschine vorteilhaft, da die Sensoreinheit hier in der Regel im Tätigkeitsbereich einer Bedienungsperson angeordnet werden muss. Im Gegensatz zu der Lösung gemäß dem älteren Vorschlag, bei dem die Messelektrode 2 in Richtung des Fasertransports beidseitig frei liegt und daher gegen äußere Störungen, z. B. Annäherungen mit der Hand, vergleichsweise empfindlich ist, ist die erfindungsgemäße Sensoreinheit gegenüber derartigen Störungen weitgehend unempfindlich. Außerdem wird das Fasermaterial 1 im Gegensatz zum älteren Vorschlag nicht über einen Sensorkopf, der aus mehreren Teilen, wie z. B. je einer Messelektrode, Schirmelektrode und Isolatorschicht zusammengesetzt ist, sondern über eine einteilig herstellbare Sendeelektrode 3 geführt, wodurch die Gefahr, dass sich das Fasermaterial 1 in der Sensoreinheit klemmt oder verhakt, beseitigt ist.

Auch im Hinblick auf ihren konstruktiven Aufbau bietet die erfindungsgemäße Lösung Vorteile. Wie insbesondere Fig. 2 zeigt, kann nach dem Durchstecken des Koaxialkabels 6 durch eine Bohrung der Schirmelektrode 4 zunächst dessen Innenleiter 6a längs einer Lötstelle 15 mit der Messelektrode 2 und nach deren Anordnung im Aufnahmeraum 12 der Außenleiter 6b längs einer Lötstelle 16 mit der Schirmelektrode 4 verbunden werden. Im Übrigen wird die Anordnung vorzugsweise so getroffen, dass die zwischen der Sendeelektrode 3 und der Schirmelektrode 4 verlaufenden Feldlinien auch die Messfläche 2a durchsetzen.

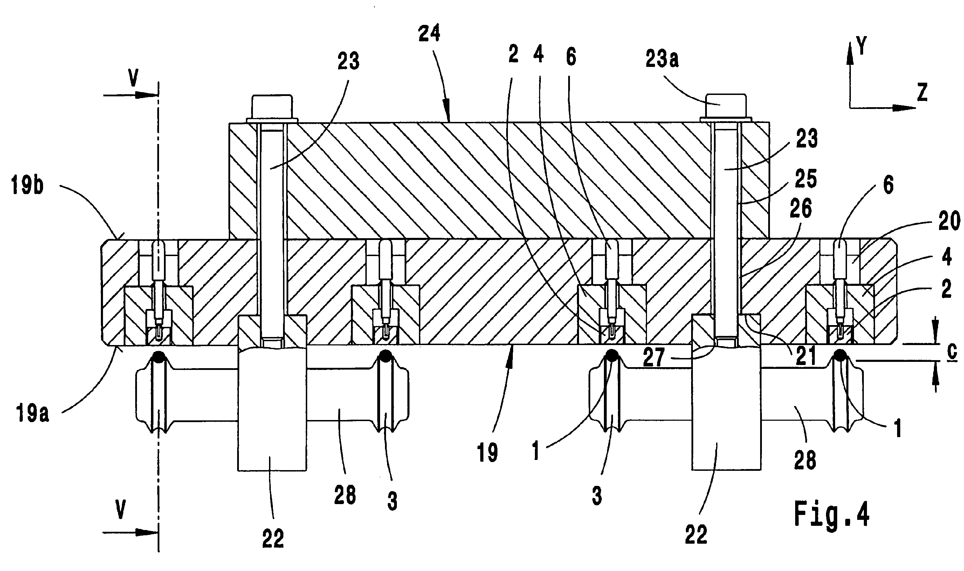

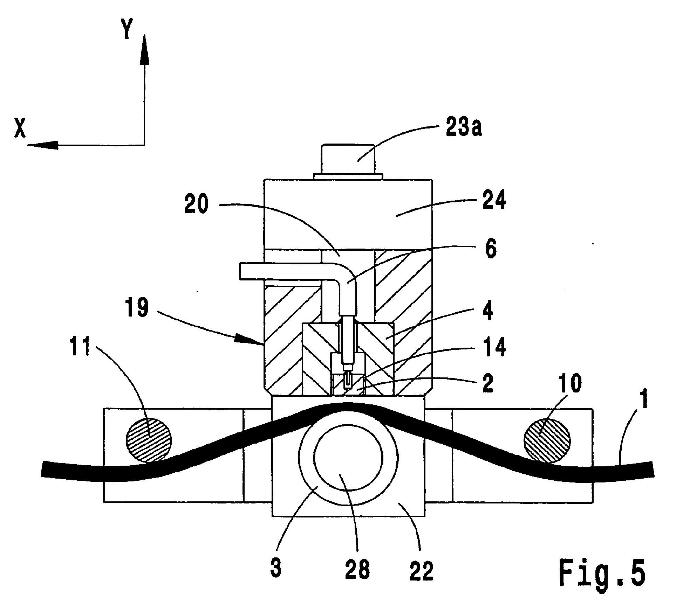

Fig. 4 und 5 zeigen ein für die praktische Anwendung besonders gut und derzeit für am besten gehaltenes Ausführungsbeispiel der erfindungsgemäßen Sensoreinheit. Diese enthält einen aus einem elektrischen Isoliermaterial bestehenden Sensorhalter 19, der hier als eine aus einem massiven Kunststoffkörper bestehende Sensorbank ausgebildet ist, in der quer zur Transportrichtung des Fasermaterials 1 mehrere, vorzugsweise identisch ausgebildete Elektrodenanordnungen nach Fig. 1 bis 3 nebeneinander angeordnet sind. Im Ausführungsbeispiel sind insgesamt vier Elektrodenanordnungen vorgesehen, von denen nachfolgend nur eine beschrieben wird.

Gemäß Fig. 4 und 5 ist die Schirmelektrode 4 und mit ihr die Messelektrode 2 in einer Aussparung 20 des Sensorhalters 19 angeordnet und bis zu einem durch eine Stufe gebildeten Anschlag in diese eingesetzt. Die Anordnung ist derart gewählt, dass die Messfläche 2a und die Stirnfläche 4a dadurch mit einer ebenen Unterseite 19a des Sensorhalters 19 bündig abschließen. Durch einen entgegengesetzt dazu angeordneten Abschnitt der Aussparung 20 wird das Koaxialkabel 6 nach außen geführt.

Der Sensorhalter 19 ist außerdem mit einer an seiner Unterseite endenden Aussparung 21 versehen, in die ein über die Unterseite 19a vorstehender Montageklotz 22 eingesetzt ist. Der Montageklotz 22 ist mittels einer Befestigungsschraube 23 an einem Haltekörper 24 befestigt, der auf einer entgegengesetzt zur Unterseite 19a liegenden Oberseite 19b des Sensorhalters 19 angeordnet ist. Die Befestigungsschraube 23 durchragt koaxiale Bohrungen 25 und 26 im Haltekörper 24 und Sensorhalter 19 und wird in eine mit diesen koaxiale Gewindebohrung 27 des Montageklotzes 22 eingedreht, bis ihr Kopf 23a am Haltekörper 24 anliegt und sowohl der Sensorhalter 19 als auch der Haltekörper 24 zwischen dem Kopf 23a und dem Montageklotz 22 fest verspannt sind.

Der Montageklotz 22 weist eine parallel zur Unterseite 19a des Sensorhalters 19, d. h. parallel zur z-Richtung des gedachten Koordinatensystems erstreckte Bohrung auf, in der eine Stange 28 befestigt ist, die im Ausführungsbeispiel auf beiden Seiten des Montageklotzes 22 aus diesem herausragt. An den Stirnseiten dieser Stange 28 ist je eine der Sendelektroden 3 befestigt, über die entsprechend Fig. 1 und 2 das in X-Richtung transportierte Fasermaterial 1 geführt wird. Die Sendeelektroden 3 sind an ihrem Umfang vorzugsweise leicht exzentrisch ausgebildet und in der Stirnseite der Stange 28 drehbar gelagert. Dadurch kann ihr Abstand c (vgl. Fig. 1) von der Messelektrode 2 auf einfache Weise eingestellt und an die jeweiligen Bedürfnisse angepasst werden. Alternativ können natürlich auch andere Mittel vorgesehen sein, um die Lage der Sendelektrode 3 relativ zur Messfläche 2a der Messelektrode 2 einzustellen.

Im beschriebenen Ausführungsbeispiel bestehen der Montageklotz 22, der Haltekörper 24, die Befestigungsschraube 23, die Stange 28 und die Sendeelektroden 3 aus elektrisch gut leitenden Materialien. Dadurch ergibt sich der Vorteil, dass die Sendeelektrode 3, wenn die beschriebene Sensoreinheit mittels des Haltekörpers 24 an einem ebenfalls elektrisch gut leitenden Bauteil einer Rundstrickmaschine, eines Streckwerks od. dgl. befestigt wird, automatisch das Potential der Maschinenmasse annimmt, wie in Fig. 1 angedeutet ist, d. h. elektrisch an Masse liegt, ohne dass hierzu weitere Maßnahmen erforderlich sind.

Im Übrigen versteht sich, dass beim Ausführungsbeispiel nach Fig. 4 einerseits zwei Sendeelektroden 3 an der Stange 28 befestigt sind und andererseits der Sensorhalter 19 mit einer zweiten, entsprechend ausgebildeten Einheit versehen ist, die einen weiteren am Haltekörper 24 befestigten Montageklotz 22 mit einer weiteren Stange 28 und zwei weiteren, an dieser angebrachten Sendeelektroden 3 enthält. Allerdings ist klar, dass die Anordnung auch so gewählt werden könnte, dass der Sensorhalter 19 nur mit einer einzigen Einheit oder mit mehr als zwei Einheiten aus Montageklotz 22, Stange 28 und Sendeelektrode 3 versehen werden könnte. Außerdem könnte jede Einheit mit nur einer Sendeelektrode 3 versehen sein.

Die in Fig. 5 dargestellten Niederhalter 10 und 11 sind vorzugsweise an in Y-Richtung erstreckten Verlängerungen des Montageklotzes 22 befestigt und als über die ganze Länge des Sensorhalters 19 erstreckte Stangen ausgebildet. Dadurch wird für die hier vier geführten Fasermaterialien 1 nur je ein gemeinsamer Niederhalter 10 bzw. 11 benötigt.

Die beschriebene Ausbildung der Sensoreinheit bringt den Vorteil mit sich, dass sie lediglich an einem auf Maschinenmasse liegenden Bauteil befestigt werden braucht und nach dem Anlegen der Generatorspannung zwischen die Schirmelektrode 4 und die Sendeelektrode 3 voll funktionsfähig ist, ohne dass komplizierte Einstellarbeiten erforderlich sind.

Zur Inbetriebnahme der Sensorseinheit nach dem 3-Elektroden-Messprinzip wird mittels des Generators 5 ein Anregungsfeld zwischen der Sendeelektrode 3 (Maschinenmasse) und der Schirmelektrode 4 eingespeist, wobei die Messelektrode 2 zwischen diesen beiden eingeprägten Potentialen angeordnet und über das Fasermaterial 1 an das elektrische Feld angekoppelt ist. Die Messelektrode 2 befindet sich außerdem auf einem schwimmenden Potential. Das über die Sendeelektrode 3 gleitende Fasermaterial 1 bewirkt daher beim Auftreten von Inhomogenitäten (Fehlstellen) eine Veränderung des elektrischen Feldes und damit auch eine Potentialänderung an der Messelektrode 2. Durch die beschriebene Gestaltung der Sendeelektrode 3 und der Schirmelektrode 4 und weitere Optimierungen der Sensorgeometrie kann die erzielbare Signalstärke des Messsignals weiter verbessert werden. Dies gelingt insbesondere dann, wenn das vom Generator 5 erzeugte Anregungsfeld (bzw. der Feldlinienverlauf) entsprechend der obigen Beschreibung auf die Messelektrode 2 konzentriert und für eine intensive Durchflutung des Fasermaterials 1 gesorgt wird. Mit anderen Worten sollte der Feldanteil, der nicht das Fasermaterial 1 durchflutet, möglichst klein oder zumindest konstant gehalten werden. In der erfindungsgemäßen Anordnung wird bewirkt, dass im Randbereich der Messelektrode 2 relativ konstante Bedingungen herrschen und Wärmebewegungen, Temperaturdriften der Isolationsmaterialien, insbesondere deren Änderungen der Dielektrizitätskonstante, nicht zu Potentialänderungen der Messelektrode 2 führen. Außerdem hat es sich als zweckmäßig erwiesen, den Abstand zwischen der Sendeelektrode 3 und der Messelektrode 2 möglichst klein zu wählen. Dadurch wird eine hohe Feldstärke des Anregungsfeldes bewirkt, die unmittelbar die Messsteilheit positiv beeinflusst.

Der Verstärker 7 und zumindest Teile der Auswerteelektronik 8 für die von der Messelektrode 2 abgegebenen Messsignale können vorzugsweise ebenfalls in der Sensoreinheit bzw. im Sensorhalter 19 untergebracht werden. In diesem Fall wird das Messsignal z. B. mit Hilfe von Verstärkern auf einen solchen Signalpegel angehoben, dass es anschließend unter Verzicht auf Koaxialkabel frei von Störbeeinflussungen mit einfachen Kabeln zur Auswerteelektronik 8 weitergeleitet werden kann.

Die Auswertung der Messsignale kann in an sich beliebiger Weise erfolgen, wird bevorzugt aber mit Hilfe von Schaltungsanordnungen durchgeführt, die speziell für das kapazitive 3-Elektroden-Messprinzip entwickelt wurden und zur Detektion extrem kleiner Kapazitätsänderungen geeignet sind. Insbesondere wird in diesem Zusammenhang auf eine Auswertetechnik verwiesen, die aus der Druckschrift DE 100 27 507 C1 bekannt ist.

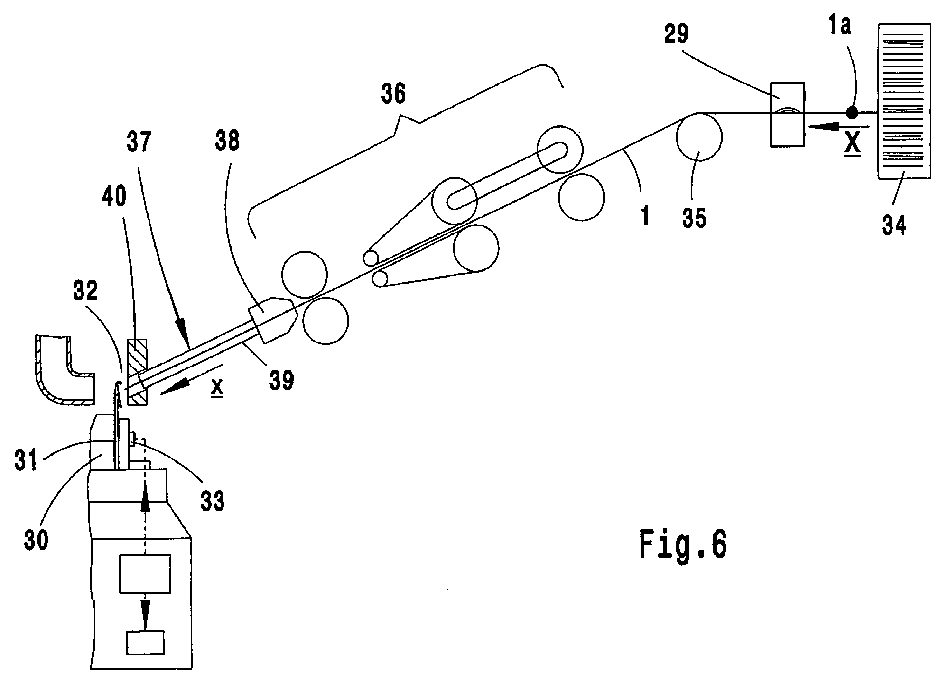

Fig. 6 zeigt eine Teilansicht einer Maschine zur Herstellung von Maschenware am Beispiel einer Rundstrickmaschine mit einer Vielzahl von Maschenbildungsstellen (Stricksystemen), denen wenigstens je eine der beschriebenen, hier mit dem Bezugszeichen 29 bezeichneten Sensoreinheiten zugeordnet ist.

Die Rundstrickmaschine enthält einen Nadelzylinder 30, in dem übliche Stricknadeln 31 verschiebbar gelagert sind, die an einer nachfolgend als Stricksystem 32 bezeichneten Maschenbildungsstelle mit Hilfe von nicht näher dargestellten Schlossteilen 33 in eine zur Aufnahme des Fasermaterials 1 geeignete Faseraufnahmestellung bewegt werden können. Das Fasermaterial 1 wird der Rundstrickmaschine von Vorratsbehältern 34 wie z. B. Kannen, Vorratsspulen od. dgl. aus zugeführt.

Das Fasermaterial 1 wird über ein nicht dargestelltes Transportmittel und ggf. eine Umlenkrolle 35 einem Streckwerk 36 zugeführt. Jedem der Stricksysteme 32, von denen in Fig. 6 nur eines dargestellt ist, ist ein derartiges Streckwerk 36 zugeordnet, das in an sich bekannter Weise z. B. drei oder mehr Paare von Streckwalzen aufweist.

Das aus dem Streckwerk 36 kommende Fasermaterial 1 wird in bekannter Weise vorzugsweise mit Hilfe einer allgemein mit dem Bezugszeichen 37 bezeichneten Spinn- bzw. Transportvorrichtung einem zugeordneten Stricksystem 32 zugeführt. Die Transportvorrichtung 37 enthält z. B. ein Drallorgan 38 und ein an dieses angeschlossenes Spinn- bzw. Transportrohr 39, das an einem Fadenführer 40 endet, der wie üblich dicht vor den Stricknadeln 31 und so angeordnet ist, dass das Fasermaterial 1 in die Haken der Stricknadeln 31 eingelegt wird.

Rundstrickmaschinen der beschriebenen Art sind z. B. aus der Druckschrift PCT WO 2004/079068 bekannt.

Ziel der Erfindung ist es, das Auftreten der eingangs erwähnten Fehlstellen in der fertigen Maschenware zu verhindern. Zu diesem Zweck wird erfindungsgemäß zunächst ganz allgemein vorgeschlagen, das Fasermaterial 1 vor seiner Ankunft an einem Stricksystem 32 auf die erforderlichen Qualitätsmerkmale, insbesondere Dicke- und Masseschwankungen zu überprüfen, und beim Erkennen von Fasermaterialabschnitten, die sich durch unzulässige Abweichungen von einer vorgewählten Qualität auszeichnen, zu verhindern, dass diese fehlerhaften Fasermaterialabschnitte in die Stricknadeln 31 eingelegt werden. Dies wird dadurch erreicht, dass das Fasermaterial 1 mit Hilfe der entsprechend Fig. 1 bis 5 ausgebildeten Sensoreinheit 29 überwacht wird und beim Erkennen einer Fehlstelle, die in Fig. 6 schematisch durch einen Punkt 1a angedeutet ist, der Maschenbildungsprozess unterbrochen, die Fehlstelle 1a aus dem Fasermaterial 1 entfernt und der Maschenbildungsprozess dann fortgesetzt wird. Die Unterbrechung des Maschenbildungsprozesses kann z. B. dadurch erfolgen, dass die Strickwerkzeuge 31 am betreffenden Stricksystem 32 ab Erkennung einer Fehlstelle 1a ohne Abwerfen zuvor gebildeter Maschen und ohne Aufnahme von Fasermaterial 1 am Stricksystem 32 vorbeigeführt und zu diesem Zweck z. B. in eine Rundlaufbahn gesteuert werden. Die Sensoreinheit 29 kann entsprechend Fig. 6 in Transportrichtung X vor dem Streckwerk 36 oder auch an einer anderen, vor dem betreffenden Stricksystem 32 liegenden Stelle angeordnet sein.

Die Erfindung ist nicht auf die beschriebenen Ausführungsbeispiele beschränkt, die auf vielfache Weise abgewandelt werden können. Das gilt insbesondere für die oben angegebenen Maße für die verschiedenen Krümmungsradien, Abstände und sonstigen Abmessungen, da diese Maße u. a. auch von der Art und den Dimensionen des untersuchten Fasermaterials 1 abhängen. Aus demselben Grund kann es zweckmäßig sein, der Gleitfläche 9 einen mehr kreisförmigen Querschnitt (Fig. 2) oder einen mehr quadratischen oder rechteckigen Querschnitt zugeben. Außerdem ist klar, dass die Messflächen 2a der Messelektroden 2, wenn Lunten mit leitfähigen Anteilen, z. B. in Form von Metallfäden verwendet werden, mit einer dünnen Isolationsschicht versehen sein sollten, um elektrische Kurzschlüsse zu vermeiden. Ferner umfasst die Erfindung nicht nur Maschinen zur Herstellung von Maschenware, die entsprechend Fig. 6 ausgebildet sind, sondern auch z. B. solche, bei denen die Verfeinerung bzw. Verstreckung des Fasermaterials nicht mit Hilfe von Streckwerken, sondern auf andere Weise erfolgt (z. B. PL 350 489 A).

## Patentansprüche

1. Kapazitiv arbeitende Sensoreinheit zur Überwachung der Qualität von in einer Transporteinrichtung bewegtem Fasermaterial (1), enthaltend eine Elektrodenanordnung, die eine erste, eine zweite und eine dritte, jeweils aus einem elektrisch leitenden Material bestehende Elektrode (3, 2, 4) und einen von der ersten und der zweiten Elektrode begrenzten Durchgang für das Fasermaterial (1) aufweist, wobei die erste Elektrode eine Sendeelektrode (3) mit einer Oberseite ist, die eine in Transportrichtung erstreckte Gleitfläche (9) für das Fasermaterial (1) enthält, dass die zweite Elektrode eine Messelektrode (2) mit einer der Oberseite der Sendeelektrode (3) mit Abstand gegenüberstehenden Messfläche (2a) ist und dass die dritte Elektrode eine die Messelektrode (2) umgebende Schirmelektrode (4) ist, **dadurch gekennzeichnet, dass** die Messelektrode (2) bis auf den Bereich der Messfläche (2a) von der Schirmelektrode (4) umgeben ist.

2. Sensoreinheit nach Anspruch 1, **dadurch gekennzeichnet, dass** die Sendeelektrode (3) eine in Transportrichtung bogenförmig gekrümmte, konvexe Oberseite aufweist.

3. Sensoreinheit nach Anspruch 2, **dadurch gekennzeichnet, dass** die Gleitfläche (9) muldenförmig mit einer Tiefe (a) ausgebildet ist.

4. Sensoreinheit nach einem der Ansprüche 1 bis 3, **dadurch gekennzeichnet, dass** die Sensoreinheit eine Eintritts- und eine Austrittsseite für das Fasermaterial aufweist, wobei die Eintrittes- und die Austrittsseite der Sensoreinheit je einen zur Einwirkung auf das Fasermaterial (1) bestimmten, eine gleichförmige flächige Auflage des Fasermaterials (1) auf der Gleitfläche (9) erzwingenden Niederhalter (10, 11) aufweist.

5. Sensoreinheit nach einem der Ansprüche 1 bis 4, **dadurch gekennzeichnet, dass** sie einen eine Baueinheit bildenden, die Messelektrode (2) und die Schirmelektrode (4) aufweisenden Sensorkopf enthält.

6. Sensoreinheit nach Anspruch 5, **dadurch gekennzeichnet, dass** die Schirmelektrode (4) topfförmig ausgebildet und mit einem Aufnahmeraum (12) versehen ist und dass die Messelektrode (2) in dem Aufnahmeraum (12) angeordnet und durch eine Isolationsschicht (14) elektrisch von der Schirmelektrode (4) isoliert ist.

7. Sensoreinheit nach Anspruch 6, **dadurch gekennzeichnet, dass** die Schirmelektrode (4) eine bündig mit der Messfläche (2a) abschließende Stirnfläche (4a) aufweist.

8. Sensoreinheit nach Anspruch 6 oder 7, **dadurch gekennzeichnet, dass** die Messelektrode (2) mit einem zum Anschluss an eine Auswerteelektronik (8) bestimmten Innenleiter(6a) eines Koaxialkabels (6) verbunden ist, dessen Außenleiter (6b) an die Schirmelektrode (4) angeschlossen ist.

9. Sensoreinheit nach Anspruch 8, **dadurch gekennzeichnet, dass** der Innenleiter (6a) in einem von der Messfläche (2a) abgewandten Abschnitt des Aufnahmeraums (12) mit der Messelektrode (4) verbunden ist.

10. Sensoreinheit nach einem der Ansprüche 6 bis 9, **dadurch gekennzeichnet, dass** die Schirmelektrode (4) in einem aus Isoliermaterial bestehenden Sensorhalter (19) angeordnet ist.

11. Sensoreinheit nach Anspruch 10, **dadurch gekennzeichnet, dass** die Sendeelektrode (3) an einem mit dem Sensorhalter (19) verbundenen Haltekörper (24) befestigt ist.

12. Sensoreinheit nach Anspruch 11, **dadurch gekennzeichnet, dass** die Sendeelektrode (3) in Richtung der Messfläche (2a) verstellbar am Haltekörper (24) befestigt ist.

13. Sensoreinheit nach einem der Ansprüche 2 bis 12, **dadurch gekennzeichnet, dass** ein Abstand (c) der Messelektrode (21) von einem höchsten Punkt der Sendeelektrode (18) eine Größe besitzt, die höchsten einem Ein- bis Zweifachen des Durchmessers des Fasermaterials entspricht.

14. Sensoreinheit nach einem der Ansprüche 3 bis 13, **dadurch gekennzeichnet, dass** die Tiefe (a) der Gleitfläche (9) höchstens gleich dem Durchmesser des Fasermaterials (1) ist.

15. Sensoreinheit nach einem der Ansprüche 1 bis 14, **dadurch gekennzeichnet, dass** die Sendeelektrode (3) elektrisch an Masse liegt.

16. Sensoreinheit nach einem der Ansprüche 1 bis 15, **dadurch gekennzeichnet, dass** sie einen Wechselspannungs-Generator (5) zum Anlegen einer hochfrequenten Wechselspannung zwischen die Sendeelektrode (3) und die Schirmelektrode (4) aufweist.

17. Maschine zur Herstellung von Maschenware mit wenigstens einer Maschenbildungsstelle (32), Mitteln (36, 37) zur Zuführung von Fasermaterial (1) zur Maschenbildungsstelle (32) und einer Sensoreinheit (29) zur Überwachung der Qualität des Fasermaterials (1), **dadurch gekennzeichnet, dass** die Sensoreinheit (29) nach wenigstens einem der Ansprüche 1 bis 16 ausgebildet ist.

## Claims

1. Capacitively operating sensor unit for monitoring the quality of fibre material (1) which is moved in a transport direction, comprising an electrode arrangement which has a first, a second and a third electrode (3, 2, 4) which respectively consist of an electrically conducting material, and a passage for the fibre material (1) which is delimited by the first and the second electrode, the first electrode being a transmitter electrode (3) with an upper side which comprises a sliding surface (9), which extends in the transport direction, for the fibre material (1), the second electrode being a measuring electrode (2) with a measuring surface (2a) which is opposite the upper side of the transmitter electrode (3) at a spacing, and the third electrode being a screening electrode (4) which surrounds the measuring electrode (2), **characterised in that** the measuring electrode (2) is surrounded by the screening electrode (4) apart from the region of the measuring surface (2a).

2. Sensor unit according to claim 1, **characterised in that** the transmitter electrode (3) has a convex upper side which is curved arcuately in the transport direction.

3. Sensor unit according to claim 2, **characterised in that** the sliding surface (9) has a trough-shaped configuration with a depth (a).

4. Sensor unit according to one of the claims 1 to 3, **characterised in that** the sensor unit has an entry- and an exit-side for the fibre material, the entry- and exit-side of the sensor unit respectively having a holding-down clamp (10, 11), which is intended for acting on the fibre material (1) and forces a uniform planar placing of the fibre material (1) on the sliding surface (9).

5. Sensor unit according to one of the claims 1 to 4, **characterised in that** it comprises a sensor head which forms a constructional unit and has the measuring electrode (2) and the screening electrode (4).

6. Sensor unit according to claim 5, **characterised in that** the screening electrode (4) has a pot-shaped configuration and is provided with a receiving chamber (12), and **in that** the measuring electrode (2) is disposed in the receiving chamber (12) and is insulated electrically from the screening electrode (4) by an insulation layer (14).

7. Sensor unit according to claim 6, **characterised in that** the screening electrode (4) has an end-face (4a) which terminates flush with the measuring surface (2a).

8. Sensor unit according to claim 6 or 7, **characterised in that** the measuring electrode (2) is connected to an inner conductor (6a) of a coaxial cable (6) which is intended for connection to an evaluation electronic unit (8), the outer conductor (6b) of which coaxial cable is connected to the screening electrode (4).

9. Sensor unit according to claim 8, **characterised in that** the inner conductor (6a) is connected to the measuring electrode (4) in a portion of the receiving chamber (12) which is orientated away from the measuring surface (2a).

10. Sensor unit according to one of the claims 6 to 9, **characterised in that** the screening electrode (4) is disposed in a sensor holder (19) which consists of insulation material.

11. Sensor unit according to claim 10, **characterised in that** the transmitter electrode (3) is attached to a holding element (24) which is connected to the sensor holder (19).

12. Sensor unit according to claim 11, **characterised in that** the transmitter electrode (3) is attached displaceably on the holding element (24) in the direction of the measuring surface (2a).

13. Sensor unit according to one of the claims 2 to 12, **characterised in that** a spacing (c) of the measuring electrode (21) from a highest point of the transmitter electrode (18) has a size which corresponds at most to a multiple of once to twice the diameter of the fibre material.

14. Sensor unit according to one of the claims 3 to 13, **characterised in that** the depth (a) of the sliding surface (9) is at most equal to the diameter of the fibre material (1).

15. Sensor unit according to one of the claims 1 to 14, **characterised in that** the transmitter electrode (3) is electrically earthed.

16. Sensor unit according to one of the claims 1 to 15, **characterised in that** it has an alternating voltage generator (5) for applying a high-frequency alternating voltage between the transmitter electrode (3) and the screening electrode (4).

17. Machine for the production of knitwear having at least one knitting position (32), means (36, 37) for supplying fibre material (1) to the knitting position (32) and a sensor unit (29) for monitoring the quality of the fibre material (1), **characterised in that** the sensor unit (29) is configured according to at least one of the claims 1 to 16.

## Revendications

1. Unité de détection à fonctionnement capacitif pour surveiller la qualité d'un matériau fibreux (1) déplacé dans un dispositif de transport, contenant un agencement d'électrodes qui présente une première, une deuxième et une troisième électrode (3, 2, 4) constituées chacune d'un matériau électroconducteur et un passage pour le matériaux fibreux (1) limité par la première et la deuxième électrode, la première électrode étant une électrode émettrice (3) avec une face supérieure qui comporte une surface de glissement (9) s'étendant dans la direction de transport pour le matériaux fibreux (1), la deuxième électrode étant une électrode de mesure (2) avec une surface de mesure (2a) qui fait face, à distance, à la face supérieure de l'électrode émettrice (3) et la troisième électrode étant une électrode écran (4) entourant l'électrode de mesure (2), **caractérisée en ce que** l'électrode de mesure (2) est entourée par l'électrode écran (4) excepté dans la zone de la surface de mesure (2a).

2. Unité de détection selon la revendication 1, **caractérisée en ce que** l'électrode émettrice (3) présente une face supérieure convexe, courbée en forme d'arc dans la direction de transport.

3. Unité de détection selon la revendication 2, **caractérisée en ce que** la surface de glissement (9) est réalisée en forme de cuvette d'une profondeur (a).

4. Unité de détection selon l'une des revendications 1 à 3, **caractérisée en ce que** l'unité de détection présente un côté entrée et un côté sortie pour le matériau fibreux, le côté entrée et le côté sortie de l'unité de détection présentant chacun un rouleau presseur (10, 11) destiné à agir sur le matériau fibreux (1), qui impose un appui plan uniforme du matériau fibreux (1) sur la surface de glissement (9).

5. Unité de détection selon l'une des revendications 1 à 4, **caractérisée en ce qu'**elle contient une tête de détection formant une unité constructive, présentant l'électrode de mesure (2) et l'électrode écran (4).

6. Unité de détection selon la revendication 5, **caractérisée en ce que** l'électrode écran (4) est réalisée en forme de pot et pourvue d'un espace de réception (12) et que l'électrode de mesure (2) est disposée dans l'espace de réception (12) et isolée électriquement de l'électrode écran (4) par une couche isolante (14).

7. Unité de détection selon la revendication 6, **caractérisée en ce que** l'électrode écran (4) présente une surface frontale (4a) se terminant en affleurement avec la surface de mesure (2a).

8. Unité de détection selon la revendication 6 ou 7, **caractérisée en ce que** l'électrode de mesure (2) est reliée à un conducteur intérieur (6a) d'un câble coaxial (6) destiné à la connexion à une électronique d'évaluation (8), dont le conducteur extérieur (6b) est connecté à l'électrode écran (4).

9. Unité de détection selon la revendication 8, **caractérisée en ce que** le conducteur intérieur (6a) est relié à l'électrode de mesure (4) dans une partie de l'espace de réception (12) éloignée de la surface de mesure (2a).

10. Unité de détection selon l'une des revendications 6 à 9, **caractérisée en ce que** l'électrode écran (4) est disposée dans un support de capteur (19) en matériau isolant.

11. Unité de détection selon la revendication 10, **caractérisée en ce que** l'électrode émettrice (3) est fixée à un corps de support (24) relié au support de capteur (19).

12. Unité de détection selon la revendication 11, **caractérisée en ce que** l'électrode émettrice (3) est fixée au corps de support (24) de manière réglable dans la direction de la surface de mesure (2a).

13. Unité de détection selon l'une des revendications 2 à 12, **caractérisée en ce qu'**une distance (c) de l'électrode de mesure (21) à un point culminant de l'électrode émettrice (18) possède une grandeur qui correspond au plus à une à deux fois le diamètre du matériau fibreux.

14. Unité de détection selon l'une des revendications 3 à 13, **caractérisée en ce que** la profondeur (a) de la surface de glissement (9) est au plus égale au diamètre du matériau fibreux (1).

15. Unité de détection selon l'une des revendications 1 à 14, **caractérisée en ce que** l'électrode émettrice (3) est reliée électriquement à la masse.

16. Unité de détection selon l'une des revendications 1 à 15, **caractérisée en ce qu'**elle présente un générateur de tension alternative (5) pour appliquer une tension alternative à haute fréquence entre l'électrode émettrice (3) et l'électrode écran (4).

17. Machine pour la fabrication d'articles en maille comprenant au moins un poste de formation des mailles (32), des moyens (36, 37) pour amener un matériau fibreux (1) au poste de formation des mailles (32) et une unité de détection (29) pour surveiller la qualité du matériau fibreux (1), **caractérisée en ce que** l'unité de détection (29) est réalisée selon au moins l'une des revendications 1 à 16.
